# EUROPEAN PATENT SPECIFICATION

(11) **EP 1 106 602 B1**
(45) Date of publication and mention of the grant of the patent: **27.08.2008**
(21) Application number: 99309901.9
(22) Date of filing: 09.12.1999
(51) Int. Cl.: C07C 227/40

(54) **Simulated moving bed chromatographic purification of amino acids**
Reinigung von Aminosäuren durch chromatographische simulierte Wanderbetttrennung
Purification chromatographiques en lit mobile simulé d'acides aminés

(43) Date of publication of application: 13.06.2001
(60) Divisional application: 04024282.8
(73) Proprietor: ARCHER-DANIELS-MIDLAND COMPANY, Decatur, Illinois 62526 (US)
(72) Inventor: Binder, Thomas P., Decatur, Illinois 62522 (US)
(74) Representative: Lee, Nicholas John

(56) References cited:
- EP-A- 0 337 440
- WO-A-95/14002
- WO-A-99/33784
- FR-A- 2 567 413
- FR-A- 2 666 996
- VAN WALSEM H J ET AL: "Simulated moving bed in the production of lysine" JOURNAL OF BIOTECHNOLOGY,NL,ELSEVIER SCIENCE PUBLISHERS, AMSTERDAM, vol. 59, no. 1-2, 17 December 1997 (1997-12-17), pages 127-132, XP004108030 ISSN: 0168-1656
- D. EUGENE REARICK ET AL.: "The behavior of amino acids in chromatographic molasses desugarization systems" PROC. SUGAR PROCESS. RES. CONF.,1996, pages 481-491, XP000864558

## Description

### Background of the Invention

### Field of the Invention

This invention relates to a novel method for the purification of basic amino acids, including lysine, by simulated moving bed chromatography with a strong cation exchange chromatographic material.

### Related Art

Efficient separation processes are crucial to the economics of large scale production of biomolecules. For example, lysine is currently produced by fermentation for use as a feed additive at a level of 250,000 metric tons per year. Prior to purification, a lysine fermentation broth contains not only lysine but also significant quantities of impurities such as particulate materials, inorganic nutrients, sugars, organic acids, and other amino acids. Purifying this amount of lysine from fermentation broths containing these impurities requires both large amounts of chemicals and large water inputs.

The primary process now in commercial use for the purification of lysine is ion exchange by column chromatography followed by crystallization. This process comprises the following steps. The fermentation broth which contains lysine sulfate at or near neutral pH is acidified to a pH of between one and three to ensure that the carboxyl group of the lysine molecule is protonated. This acidified solution is passed over a bed of strong cation exchange resin in the ammonium form displacing the ammonia to form a raffinate stream of ammonium sulfate and the other fermentation components that do not bind to the resin. After the resin is loaded with lysine a washing step with water is used to rinse out the resin. This is followed by elution with a strong ammonia solution to regenerate the resin in the ammonia form and elute the lysine as the free base amino acid. This lysine solution then has the free ammonia stripped off, and it is acidified to form lysine•HCL which is crystallized. See, *e.g.,* U.S. Patent No. 3,565, 951 (Ishida, M., et al.; Ajinomoto Company, Inc.); U.S. Patent No. 4,714,767 (Tanaka, K., et al.; Ajinomoto Company, Inc.); Kawakita, T., et al., "Selectivity Coefficient of Lysine for Strong Cation-Exchange Resins of the Ammonium Form," Agric. Biol. Chem. 53:2571-2577 (1989).

A variation of this process is to use two ion exchange steps: one with strong cation exchange resin as above followed by a second weak cation exchange resin to retain divalent metal cations. See, U.S. Patent No. 5,684,190 (Fechter, W.G., et al.; AECI Limited); Van Walsem, H.J. and Thompson, M.C., "Simulated moving bed in the production of lysine," J. Biotechnol. 59:127-132 (1997). This variation avoids the use of a final crystallization step in the commercial production of lysine.

Both of the above processes can be automated. See, *e.g.,* Rossiter, G.J., "Continuous adsorption and chromatography in the purification of fermentation products," Preparative and Process-Scale Liquid Chromatography (Edited by G. Subramanian), Ellis Horwood, New York, pages 162-223 (1991); Rossiter, G.J. and Tolbert, C.A., "Recovery of Amino Acids and Carboxylic Acids by Continuous Exchange," presented at the Annual Meeting of the American Institute of Chemical Engineers, 17-22 November, 1991, Los Angeles, California, 20 pages; Van Walsem, H.J. and Thompson, M.C., "Simulated moving bed in the production of lysine," J. Biotechnol. 59:127-132 (1997). The practice of automated batch chromatography has also been described as "continuous co-current chromatography" (Rossiter, G.J., "Continuous adsorption and chromatography in the purification of fermentation products," Preparative and Process-Scale Liquid Chromatography (Edited by G. Subramanian), Ellis Horwood, New York, pages 162-223 (1991)).

Both of the above processes use excessive amounts of water for washing the resin and excessive amounts of acid to convert the lysine to its divalent cation for the initial binding to the resin. All of this results in increased water and salt load to the waste streams being generated.

An alternative process proposed for the commercial production of lysine is to dry whole or ultrafiltered fermentation broth. See, U.S. Patent No. 5,133, 976 (Rouy, N.; Rhone-Poulenc). This process has some advantages, such as simplicity and minimal production of waste streams. However, the process necessarily restricts the nutrients that can be provided to the fermentation and produces a product with lower purity than a product purified by customary, chromatographic means. Reduced product purity is undesirable for a variety of reasons, including increased transportation costs.

As an alternative to batch chromatography, simulated moving bed chromatography has been applied in a variety of industrial applications including: the separation of normal paraffins from other hydrocarbon species (Broughton, D.B., "Molex: Case History of a Process, Chemical Engineering Progress, 64:60-65 (1968)); the separation of p-xylene from mixtures of other C₈ hydrocarbons (Thornton, D.P., Jr., "Extract p-Xylene with Parex," Hydrocarbon Processing, 151-155 (November 1970)); the separation of sugar isomers (U.S. Pat. No. 5,391,299 (Masuda, T., et al.; Organo Corporation); Hashimoto, K., et al., "Models for the Separation of Glucose/Fructose mixture using a Simulated Moving-Bed Adsorber," J. Chem. Engineering Japan 16: 400-406 (1983); Rossiter, G.J., "ISEP® a Moving Bed Contactor for Chromatographic Separations," presented at the 4thWorkshop on Preparative HPLC, 28-31 March, 1993, Salzburg, Austria, 21 pages); and the separation of phenylalanine from a fermentation broth by adsorbing the phenylalanine from the aqueous fermentation broth onto AMBERLITE XAD-7 chromatography resin and eluting with an aqueous solution of an alcohol, ketone, or ester (U.S. Patent No. 5,071,560 (McCulloch, B. et al.; UOP). See, generally, Barker, P.E. and Ganetsos, G., "Chemical and Biochemical Separations using Preparative and Large Scale Batch and Continuous Chromatography," Separation and Purification Methods 17:1-65 (1988); Gattuso, M.J., "Un procédé économique pour les substances chirales," Chim. Mag. 141:39-42 (1996).

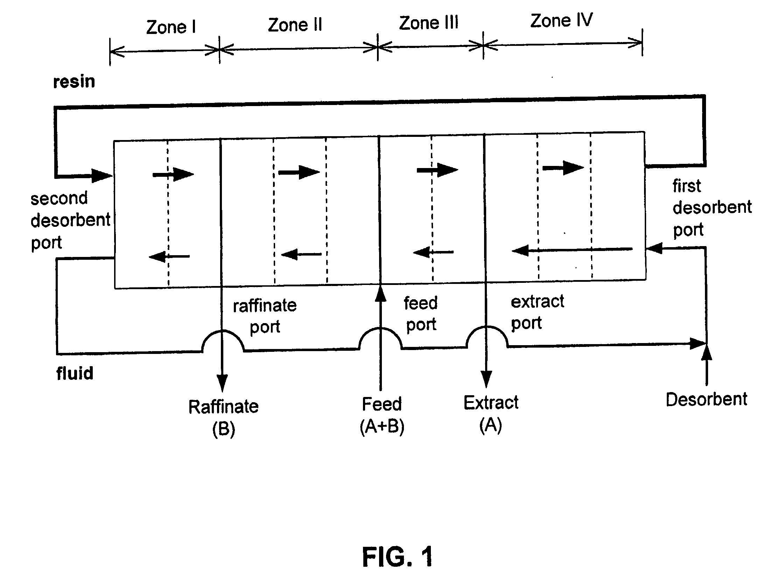

A schematic diagram of a simulated moving bed chromatographic apparatus is shown in FIG. 1. The chromatographic bed is contained within a long packed column or several columns that are connected in series. The location of the ports for the introduction or withdrawal of the desorbent, extract (= product), feed and raffinate streams are changed at given intervals to the next bed segment in the direction of the fluid flow, resulting in a simulated counter-current contact of the fluid and the resin. Simulated moving bed chromatography results in the continuous separation of two components A and B without actual movement of the resin.

The operation of a simulated moving bed chromatographic (SMBC) system has been described by S. Adachi ("Simulated moving-bed chromatography for continuous separation of two components and its application to bioreactors," J. Chromatography A 658:271-282 (1994)) as follows:
The SMBC system usually consists of four zones, I-IV, as shown schematically in Fig. 1, each of which has an inherent function. A sample solution including components A and B is fed at the feed point situated between zones II and III. Component A is adsorbed on the resin in zone II and carried into zones III and IV by the simulated movement of the resin. The absorption wave of less absorptive component B can migrate to the withdrawal point of the raffinate stream within a switching interval of the introduction and withdrawal points and component B can be recovered in the raffinate stream. Hence zone II functions for the separation of the components. Zone III is a refining zone, where component B absorbed partially in zone II is desorbed and returned to zone II. Component A adsorbed in zone II is mainly desorbed in zone IV to join the liquid stream passing through the zone, and is recovered in the extract stream. Zone I functions for adsorption of component B to suppress the dilution of component B in the raffinate stream.
Zone I may, optionally, be eliminated from the system. In the absence of Zone I, fluid flow through the raffinate stream is increased due to the elimination of the zone's terminal fluid port (i.e., second desorbent port); and the concentration of component B in the raffinate stream is correspondingly decreased.

Several types of simulated moving bed chromatographic processes are now in commercial use. These may be conceptually divided into moving port systems and moving column systems (Barker, P.E. and Deeble, R.E., "Sequential Chromatographic Equipment for the Separation of a Wide Range of Organic Mixtures," Chromatographia 8:67-79 (1975). An example of a moving port system is the Sorbex process (Universal Oil Products Incorporated (UOP)). The Sorbex process consists of a vertical column subdivided into a number of interlinked compartments and the fluid inlets and outlets of each compartment are controlled by a specially designed rotary master valve. The master valve operates like a multiport stopcock and coordinates the movement of the feed and eluant inlets and the two product outlets, thereby simulating the counter-current movement between the static packed bed and the moving fluid. A second type of moving port simulated moving bed chromatographic system is the semi-continuous counter-current refiner (SCCR). SCCR systems adopt a multicolumn approach wherein counter-current movement is simulated by continuously opening and closing the appropriate valves in the direction of the mobile phase. See, Ganetsos, G. and Barker, P.E., "Large-scale Chromatography in Industrial Processing," J. Chem. Tech. Biotechnol. 50:101-108 (1991).

An example of a moving column system is the ISEP (subsequently ISEP/CSEP) system (Advanced Separation Technologies Incorporated), which is a moving bed contactor comprising multiple chromatography columns mounted on a rotary carousel. See, U.S. Patent No. 4,522,726 (Berry, W.W., et al.; Progress Equities Incorporated); U.S. Patent No. 4,764,276 (Berry, W.W., et al.; Advanced Separation Technologies Incorporated); U.S. Patent No. 5,676,826 (Rossiter, G.J., et al.; Advanced Separation Technologies Incorporated); Rossiter, G.J. and Tolbert, C.A., "Recovery of Amino Acids and Carboxylic Acids by Continuous Exchange," presented at the Annual Meeting of the American Institute of Chemical Engineers, 17-22 November, 1991, Los Angeles, California, 20 pages.; Rossiter, G., "ISEP/CSEP: A novel Contactor for Separations," presented at the Annual Meeting of the American Chemical Society, 7-11 September 1997, Los Vegas, Nevada, abstract no. 64. The ISEP system may be used for both continuous co-current chromatography (i.e., automated batch chromatography) and for continuous counter current chromatography (i.e., simulated moving bed chromatography). See, Rossiter, G.J., "Continuous adsorption and chromatography in the purification of fermentation products," Preparative and Process-Scale Liquid Chromatography (Edited by G. Subramanian), Ellis Horwood, New York, pages 162-223 (1991); Rossiter, G.J., "ISEP® a Moving Bed Contactor for Chromatographic Separations," presented at the 4thWorkshop on Preparative HPLC, 28-31 March, 1993, Salzburg, Austria, 21 pages.

Another example of a moving column system is the ADSEP system (Illinois Water Treatment (IWT)). See, Morgart, J.R. and Graaskamp, J.M., "Continuous Process Scale Chromatography," Paper No. 230, presented at the Pittsburgh Conference on Analytical Chemistry and Applied Spectroscopy, February 22, 1988, New Orleans, Louisiana, abstract + 15 pages.

A variety of chromatographic techniques have been proposed for the commercial purification of amino acids:

U.S. Patent No. 3,565,951 (Ishida, M., et al.; Ajinomoto Company, Inc.) Discloses a method for purifying lysine from a fermentation broth also containing neutral amino acids and inorganic cations, and recovering the lysine in the form of the monohydrochloride salt, by passing the broth at a pH of approximately 2.0 over a strongly acidic cation exchange resin of the ammonium type, eluting the lysine with ammonium hydroxide solution, and crystallizing the lysine as an acid salt from the partly evaporated and acidified eluant.

U.S. Patent No. 4,663,048 (Tanaka, K., et al.; Ajinomoto Company, Inc.) discloses a method for separating a basic amino acid from its fermentation broth, or the liquid resulting from the intermediate treatment of the fermentation broth, containing high-molecular weight contaminants which entails permeating the broth, or liquids resulting from the intermediate treatment of the broth, through a semi-permeable membrane, until the basic amino acid is separated from the high-molecular weight contaminants, by passing through the membrane in the permeate; contacting the permeate with a strongly acidic cation exchange resin; and eluting the adsorbed basic amino acid from the cation exchange resin.

U.S. Patent No. 4,691,054 (Tosa, T., et al.; Ajinomoto Company, Inc.) discloses a method for separating a basic amino acid from a liquor containing the same, which entails (a) contacting the basic amino containing liquor with a strongly acidic cation exchange resin at a pH at which the basic amino acid exists in the form of a divalent cation, thereby adsorbing at least a part of the basic amino acid in the form of the divalent cation; (b) contacting said ion exchange resin with a liquor containing said basic amino acid and a neutral amino acid or an acidic amino acid at a pH at which the basic amino acid exists in the form of a monovalent cation; (c) passing an eluant through said ion exchange resin, thereby eluting the basic amino acid from the ion exchange resin; and (d) separating and recovering the basic amino acid from the eluant.

U.S. Patent No. 4,714,767 (Tanaka, K., et al.; Ajinomoto Company, Inc.) discloses a process for separating basic amino acids from a liquor containing the same using cation exchange resin adsorption and elution steps in sequence, wherein the washing water employed in said adsorption and elution steps is obtained by recycling the latter portion of a liquor discharged from a first tower of the adsorption step into an adsorption step in a subsequent cycle, or by recycling a liquid discharged from a first tower at the elution step into an elution step in a subsequent cycle.

U.S. Patent No. 4,835,309 (Jaffari, M.D., et al.; Eastman Kodak Company) discloses a method for the recovery of L-lysine using an ion exchange resin, wherein a liquid solution containing L-lysine and possibly impurities is contacted with the resin. The resin is then soaked in elutant for at least about 15 minutes. The elution of L-lysine from the resin is then continued.

U.S. Patent No. 5,071,560 (McCulloch, B., et al.; UOP) discloses the use of countercurrent simulated moving bed chromatography for the purification of phenylalanine from a fermentation broth by adsorbing the phenylalanine from the aqueous fermentation broth onto AMBERLITE XAD-7 chromatography resin and eluting with an aqueous solution of an alcohol, ketone, or ester

U.S. Patent No. 5,133,976 (Rouy, N.; Rhone-Poulenc) disclose a method for preparing a stable composition containing 35 to 48% lysine sulphate for animal nutrition whereby a lysine-producing microorganism is cultured in a culture medium and sulfuric acid or ammonium sulphate is added during culturing to convert the lysine as it is formed into lysine sulphate. After allowing the concentration of the carbon source to fall to less than 2 g/l, culturing is stopped and the resultant cultured medium is concentrated and dried to produce the final product.

U.S. Patent No. 5,279,744 (Ito, H., et al.; Ajinomoto Company, Inc.) discloses a method for purification of an amino acid which comprises contacting an amino acid solution containing impurities with an ion exchange resin to selectively adsorb the amino acid onto the resin, eluting and recovering the adsorbed amino acid whereby the resin is contacted with the amino acid solution in countercurrent continuous multiple steps during adsorption and an eluant is contacted with the adsorbed resin in countercurrent continuous multiple steps during elution.

U.S. Patent No. 5,684,190 (Fechter, W.L., et al.; AECILimited) discloses a method for recovering an amino acid such as L-lysine from an aqueous solution containing the desired amino acid and impurities, comprising the steps of: (a) passing the solution over a primary cation exchange resin to adsorb the desired amino acid onto the resin at a pH lower than the isoelectric point of the desired amino acid, (b) eluting the desired amino acid from the resin using a suitable eluant having a pH higher than the isoelectric point of the desired amino acid to produce a feed solution, (c) passing the feed solution over a secondary cation exchange resin to adsorb one or more of the impurities and to produce a second solution leaving the resin, (d) collecting the second solution until the ratio of the concentrations of a selected impurity to the desired amino acid reaches a chosen value to produce a third solution which contains a lower level of impurities than the feed solution and (e) recovering the desired amino acid from the third solution without the use of a crystallization step.

There is a need for the development of a simple purification process that produces, from a fermentation broth, a purified amino acid product, such as lysine, and generates less waste chemicals and uses less water than traditional commercial methods.

### Summary of the Invention

Unexpectedly a method for the purification of basic amino acids, including lysine, using simulated moving bed chromatography with a strong ion exchange chromatographic material has been discovered.

The present invention provides a simulated moving bed chromatography method for separating a basic amino acid from a feed solution comprising said basic amino acid and impurities, comprising:
(a) selecting a simulated moving bed chromatographic apparatus comprising one or more chromatographic columns connected in series, said column or columns containing a strong cation exchange chromatographic material comprising a functional group selected from the group consisting of sulfonates, alkylsulfonates, phenylsulfonates, alkylphenylsulfonates and mixtures thereof, and sequentially comprising a first desorbent port, an extract port, a feed port and a raffinate port; and
(b) simultaneously,
   (i) contacting, through said feed port, said feed solution with the strong cation exchange chromatographic material equilibriated with an aqueous ammonia solution;
   (ii) contacting, through said first desorbent port, a basic aqueous eluant with said strong cation exchange chromatographic material; and
   (iii) withdrawing, through said extract port, an extract solution comprising said basic amino acid and a lower percentage by dry weight of said impurities than in said feed solution.

The basic amino acid may be lysine, arginine or histidine. The feed solution may be a fermentation broth, which may be a filtered fermentation broth.

The strong cation exchange chromatographic material may be a strong cation exchange resin. The strong cation exchange chromatographic material may comprise a sulfonate.

The simulated moving bed chromatographic apparatus may further comprise a second desorbent port; a solution comprising water being withdrawn through said second desorbent port simultaneously with steps b(i)-(iii).

The basic aqueous eluant may be an aqueous ammonia solution which may comprise between 0.5% and 10% ammonia, or between 1% and 5% ammonia, by weight.

Prior to contacting at (i), the pH of said feed solution may be adjusted to a pH between 1 and 10, which may be between 2 and 7 or between 3 and 5, by the addition of an acidic or a basic material.

The pH of said extract solution at (b)(iii) may be at least one pH unit greater, and may be at least 3 pH units greater, than the pH of said feed solution at (b)(i).

### Brief Description of the Figures

FIG. 1 is a schematic diagram of the process of simulated moving bed chromatography as used for the separation of two water-soluble components, A and B. Zone I is the zone of potential water recovery, component B conceptually moves with the resin. Zone II is a zone in which component B moves with the water in an aqueous fluid, and component A conceptually moves with the resin. Zone III is a zone in which component B moves with the water in an aqueous fluid, and component A conceptually moves with the resin. Zone IV is a zone in which purified component A moves with the water in an aqueous fluid.

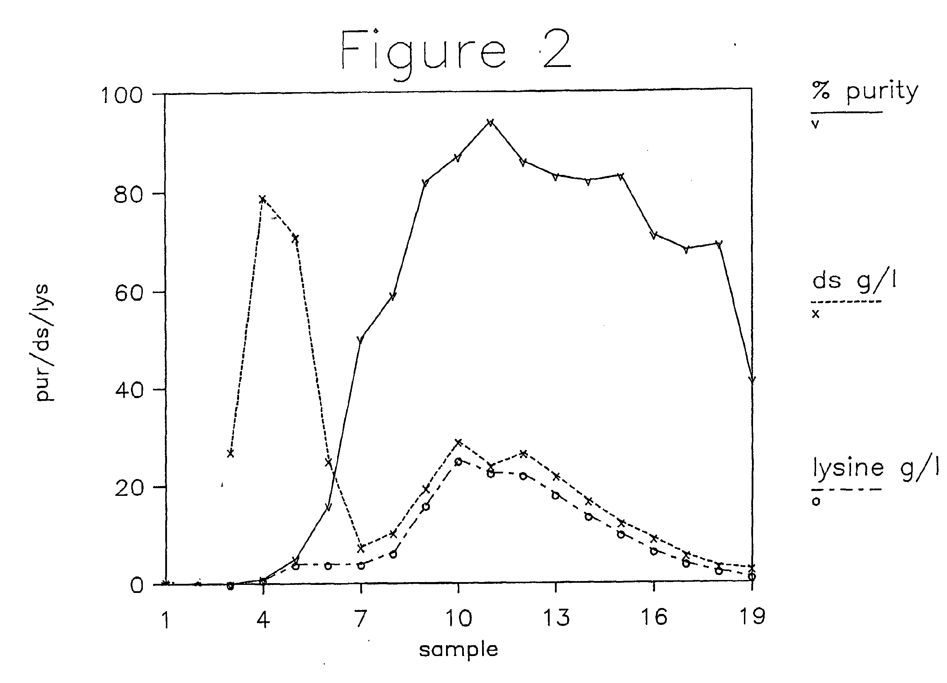

FIG. 2 is a graph showing the elution profile for lysine adsorbed onto a strong cation exchange column from a filtered fermentation broth having a pH of 9.7 and eluted with a solution of 4% aqueous ammonia at a flow rate of 2 ml/min. The graph shows the values obtained from sequential 6 ml samples of eluant analyzed for purity (v) in percent of g/l lysine free base per g/l of dry solids, dry solids (ds; x) in grams of non-volatile solids per liter, and lysine concentration (O) of each sample as lysine free base in g/l.

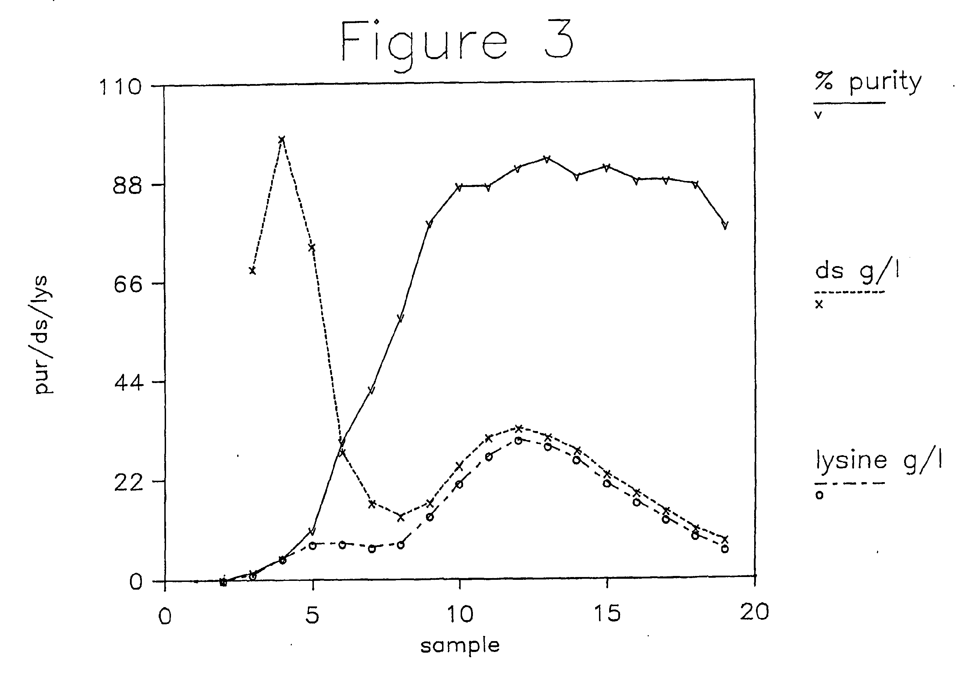

FIG. 3 is a graph showing the elution profile for lysine adsorbed onto a strong cation exchange column from a filtered fermentation broth having a pH of 8.0 and eluted with a solution of 4% aqueous ammonia at a flow rate of 2 ml/min. The graph shows the values obtained from sequential 6 ml samples of eluant analyzed for purity (v) in percent of g/l lysine free base per g/l of dry solids, dry solids (ds; x) in grams of non-volatile solids per liter, and lysine concentration (O) of each sample as lysine free base in g/l.

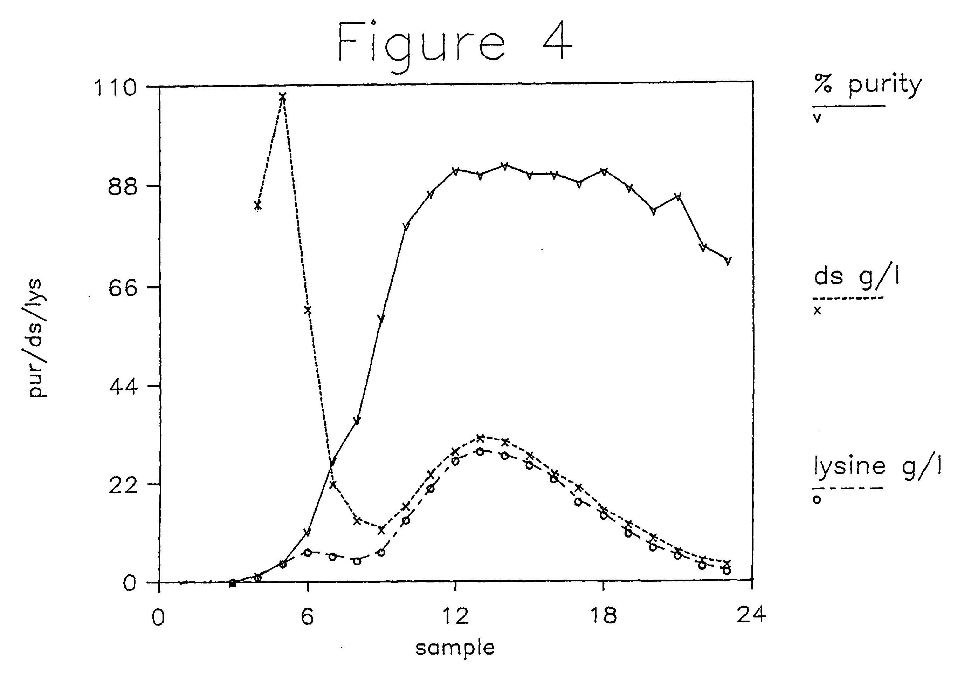

FIG. 4 is a graph showing the elution profile for lysine adsorbed onto a strong cation exchange column from a filtered fermentation broth having a pH of 4.5 and eluted with a solution of 4% aqueous ammonia at a flow rate of 2 ml/min. The graph shows the values obtained from sequential 6 ml samples of eluant analyzed for purity (v) in percent of g/l lysine free base per g/l of dry solids, dry solids (ds; x) in grams of non-volatile solids per liter, and lysine concentration (O) of each sample as lysine free base in g/l.

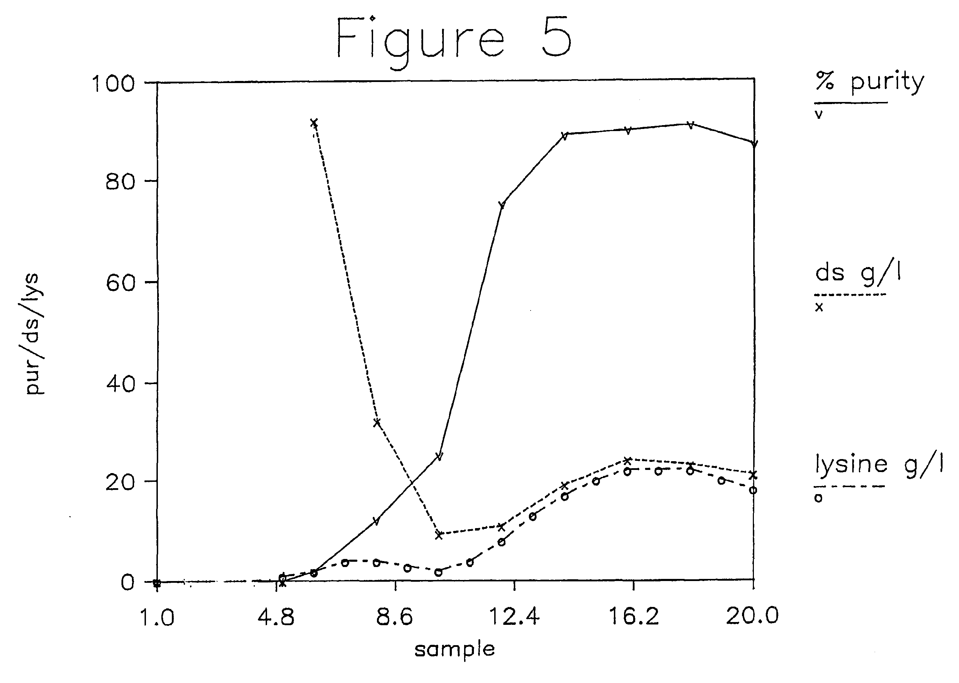

FIG. 5 is a graph showing the elution profile for lysine adsorbed onto a strong cation exchange column from a filtered fermentation broth having a pH of 4.5 and eluted with a solution of 2.61% aqueous ammonia at a flow rate of 2 ml/min. The graph shows the values obtained from sequential 6 ml samples of eluant analyzed for purity (v) in percent of g/l lysine free base per g/l of dry solids, dry solids (ds; x) in grams of non-volatile solids per liter, and lysine concentration (o) of each sample as lysine free base in g/l.

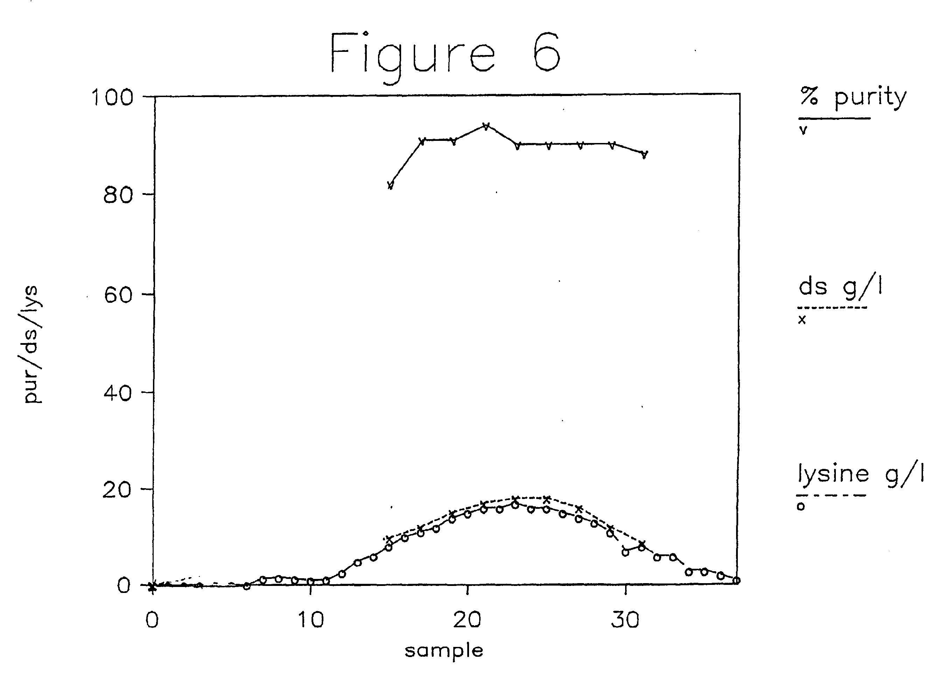

FIG. 6 is a graph showing the elution profile for lysine adsorbed onto a strong cation exchange column from a filtered fermentation broth having a pH of 4.5 and eluted with a solution of 1.58% aqueous ammonia at a flow rate of 2 ml/min. The graph shows the values obtained from sequential 6 ml samples of eluant analyzed for purity (v) in percent of g/l lysine free base per g/l of dry solids, dry solids (ds; x) in grams of non-volatile solids per liter, and lysine concentration (o) of each sample as lysine free base in g/l.

### Detailed Description of the Preferred Embodiments

The present invention relates to a simulated moving bed chromatography method for separating a basic amino acid from a feed solution comprising said basic amino acid and impurities, comprising:
(a) selecting a simulated moving bed chromatographic apparatus comprising one or more chromatographic columns connected in series, said column or columns containing a strong cation exchange chromatographic material comprising a functional group selected from the group consisting of sulfonates, alkylsulfonates, phenylsulfonates, alkylphenylsulfonates and mixtures thereof, and sequentially comprising a first desorbent port, an extract port, a feed port and a raffinate port; and
(b) simultaneously,
   (i) contacting, through said feed port, said feed solution with the strong cation exchange chromatographic material equilibriated with an aqueous ammonia solution;
   (ii) contacting, through said first desorbent port, a basic aqueous eluant with said strong cation exchange chromatographic material; and
   (iii) withdrawing, through said extract port, an extract solution comprising said basic amino acid and a lower percentage by dry weight of said impurities than in said feed solution.

The basic amino acid may be lysine, arginine or histidine. The feed solution may be a fermentation broth, which may be a filtered fermentation broth.

The strong cation exchange chromatographic material may be a strong cation exchange resin. The strong cation exchange chromatographic material may comprise a sulfonate.

The simulated moving bed chromatographic apparatus may further comprise a second desorbent port; a solution comprising water being withdrawn through said second desorbent port simultaneously with steps b(i)-(iii).

The basic aqueous eluant may be an aqueous ammonia solution which may comprise between 0.5% and 10% ammonia, or between 1% and 5% ammonia, by weight.

Prior to contacting at (i), the pH of said feed solution may be adjusted to a pH between 1 and 10, which may be between 2 and 7 or between 3 and 5, by the addition of an acidic or a basic material.

The pH of said extract solution at (b)(iii) may be at least one pH unit greater, and may be at least 3 pH units greater, than the pH of said feed solution at (b)(i).

The method of this invention may be practiced with any simulated moving bed chromatographic apparatus, including but not limited to moving port and moving column systems. Preferred are semi-continuous counter-current refiners and moving column systems. More preferred are moving column systems. Most preferred is the CSEP system (Advanced Separation Technologies, Inc.).

The strong cation exchange chromatrography material of this invention preferably comprises one or more chromatographic support materials (i.e., stationary phases). Suitable chromatographic support materials include, but are not limited to, alumina, magnesium silicates, silica, glass, controlled pore glass, carbon, porous graphitic carbon, zirconium phosphate, hydroxylapatite, calcium phosphate, magnesium carbonate, and polymers or resins. Suitable polymers or resins include, but are not limited to, hydroxyalkylmethacrolate, polyacrylamine, polymacrolate, poly(hydroxyethylmacrolate), polystyrene, styrene-divinylbenzine copolymers, poly(ethyleneglycoldimethacrolate), poly(vinylalcohol), poly(vinylacetate), and poly(vinylpyridine). Preferable are polymers or resins. More preferable are styrene-divinylbenzine copolymers.

The strong cation exchange chromatographic material of this invention further comprises a plurality of ligands, selected from one or more functional groups suitable for strong ion exchange. These functional groups include but are not limited to sulfonic acid, alkylsulfonic acid, phenylsulfonic acid, alkylphenylsulfonic acid, and salts thereof. Preferred are sulfonic acid functional groups and the salts thereof.

Specific examples of cation exchange polymers or resins that may be used include: AMBERLITE 200, AMBERLITE IR-118H, AMBERLITE IR-120PLUS, AMBERLITE IR-122, AMBERLITE IR-130C, AMBERLITE I6641, AMBERLITE IRP-69, DOWEX 50X1-100, DOWEX 50X2-100, DOWEX 50X2-200, DOWEX 50X2-400, DOWEX 50X4-100, DOWEX 50X4-200, DOWEX 50X4-200R, DOWEX 50X4-400, DOWEX 18880, DOWEX 50X8-100, DOWEX 50X8-200, DOWEX 50X8-400, DIAION 1-3561, DIAION 1-3565, DIAION 1-3570, DIAION 1-3573, DIAION 1-3577, DIAION 1-3581, DUOLITE D 5427, and DUOLITE D 5552, which are available from Sigma-Aldrich, St. Louis Missouri, U.S.A.; DIAION HPK25, DIAION PK208, DIAION PK228, DIAION SK1B, DIAION SK1BS, DIAION SK104, DIAION SK112, DIAION SK116, DOWEX HCR-S, DOWEX HCR-W2, DOWEX MSC-1, DOWEX 650C, DOWEX G-26, DOWEX 88, DOWEX MONOSPHERE 88, DOWEX MONOSPHERE 99K/320, DOWEX MONOSPHERE 99K/350, DOWEX MONOSPHERE 99Ca/320, DOWEX MONOSPHERE 99Ca/350, DOWEX Marathon C, DOWEX -032, DOWEX -406, DOWEX -437, DUOLITE C-280, and DUOLITE C-291, which are available from Supelco, Inc., Bellefonte, Pennsylvania, U.S.A.; AMBERLITE IR-120, AMBERLITE IR-120B, AMBERLITE IR-200C, AMBERLITE CG 6000, DIAION SK-1B, DOWEX XUS 40406.00, DOWEX XUS 43518, and DOWEX C500ES. Preferable are AMBERLITE IR-120, AMBERLITE IR-120B, AMBERLITE IR-200C, DOWEX C500ES, DOWEX XUS 43518, and DOWEX XUS 40406.00. Most preferable is DOWEX XUS 40406.00.

Specific examples of strong cation exchange silica-based chromatographic materials include ADSORBOSPHERE SCX, BAKERBOND SCX, PARTISIL SCX, SPHERISORB S SCX, SUPELCOSIL LC-3SCX, ULTRASIL-CX, and ZORBAX 300 SCX.

Prior to chromatography, the feed solution may be clarified by any process suitable to the production of an edible product including, but not limited to, centrifugation and filtration including ultrafiltration. Preferable is filtration, more preferable is ultrafiltration.

Prior to chromatography, the pH of the feed solution may be adjusted by the addition of any compound suitable to the production of an edible product. Suitable acidic compounds include, but are not limited to, hydrochloric acid, sulfuric acid and phosphoric acid. Preferable is sulfuric acid. Suitable basic compounds include, but are not limited to, sodium hydroxide, potassium hydroxide, ammonium hydroxide and ammonia.

The present invention is described in further detail in the following nonlimiting examples:

### Examples

### Example 1

### Effect of Feed pH on the Purification of Lysine using Column Chromatography with a Strong Cation Exchange Resin

A 60 x 1.5 cm column of IR 120 resin (Rohm and Haas) was prepared and equilibrated with 4% ammonia aqueous solution. Three 13 ml samples of ultrafiltered lysine fermentation broth, with lysine HCL equivalent of 120 g/l, at pH values of 9.7, 8.0 and 4.5, respectively, were sequentially loaded onto the column and eluted with 4% ammonia aqueous solution at a flow rate of 2 ml/min. Sequential 6 ml samples were collected and analyzed for purity (v) in percent of g/l lysine free base per g/l of dry solids, dry solids (ds; x) in grams of non-volatile solids per liter, and lysine concentration (o) of each sample as lysine free base in g/l. The results are shown in FIG. 2 (pH 9.7), FIG. 3 (pH 8.0), and FIG. 4 (pH 4.5). FIGs. 2-4 illustrate that adsorption onto a strong cation exchange resin and desorption with aqueous ammonia is effective for the separation of lysine from other dissolved materials from fermentation broths having a range of different pH values from at least as low as 4.5 to at least as high as 9.7.

### Example 2

### Effect of Eluant Ammonia Concentration on the Purification of Lysine using Column Chromatography with a Strong Cation Exchange Resin

A 60 x 1.5 cm column of IR 120 resin (Rohnm and Haas) was prepared and equilibrated with 4% ammonia aqueous solution. A 13 ml sample of ultrafiltered lysine fermentation broth, with lysine HCL equivalent of 120 g/l, at pH 4.5 was loaded onto the column and eluted with 2.61% ammonia aqueous solution at a flow rate of 2 ml/min. Sequential 6 ml samples were collected and analyzed for purity (v) in percent of g/l lysine free base per g/l of dry solids, dry solids (ds; x) in grams of non-volatile solids per liter, and lysine concentration (o) of each sample as lysine free base in g/l. The procedure was repeated with an eluant of 1.58% ammonia aqueous solution at a flow rate of 2 ml/min. The results are shown in FIG. 5 (2.61% ammonia) and FIG. 6 (1.58% ammonia). FIGs. 5 and 6 illustrate that lysine can be separated effectively from other dissolved materials from a fermentation broth by adsorption onto a strong cation exchange resin and elution with aqueous ammonia having a concentration at least as low as 1.58%.

### Example 3

### Purification of Lysine from a Fermentation Broth by Simulated Moving Bed Chromatography

A pilot simulated moving bed apparatus from Advanced Seperation Technologies with twenty 1.8 liter columns was filled with XUS 40406.00 cation exchange resin (Dow Chemical). This system was then employed in five separate trials for the purification of lysine from fermentation broths. During these trials only zones 2, 3 and 4, as diagramed schematically in FIG. 1, were used. In all trials, the feed pH was 4.5; and the simulated resin flow rate was 200 ml/min. The results from these trials are shown in Table 1.

**Table 1.Results for the Purification of Lysine from a Fermentation Broth by Simulated Moving Bed Chromatography**

| Trial | Columns | | | Flows | | | Properties | | | | |
|---|---|---|---|---|---|---|---|---|---|---|---|
| | in zone | in zone | in zone | Feed ml/min | Des. m/min | Ext. ml/min | Feed [LHCl] | Des. [NH₄] | Ext. [LHCl] | Ext. purity | Ext. yield |
| | 2 | 3 | 4 | | | | g/l | % | g/l | % | % |
| 1 | 5 | 5 | 5 | 98 | 255 | 151 | 125 | 4 | 79 | 96 | 98 |
| 2 | 4 | 3 | 6 | 140 | 170 | 172 | 120 | 6 | 95 | - | 97 |
| 3 | 6 | 5 | 5 | 114 | 250 | 134 | 89 | 4 | 61 | 97 | 99 |
| 4 | 6 | 5 | 5 | 274 | 243 | 215 | 89 | 4 | 105 | 76 | 99 |
| 5* | 5 | 4 | 6 | 95 | 228 | 123 | 126 | 3.4 | 100 | 98 | 97 |
| Des. = Desorbent | | | | | | | | | | | |
| Ex. = extract | | | | | | | | | | | |
| [LHCl] = concentration of lysine as lysine•HCl | | | | | | | | | | | |
| * This example had the first column in the zone 4 being eluted with water only at 200 ml/min with 28 ml/min of 28% ammonia being added to it as it entered the next column. In the example, the pH gradient in zone 2 was monitored. The pH ranged from 5.6 at the feed port; pH 8-6 at the midpoint and pH 10 at the extract port of zone 2, providing a gradient sufficient for purification. | | | | | | | | | | | |

The date in Table 1 indicates that, under the conditions of this example, simulated moving bead chromatography can be used effectively for the purification of lysine from a fermentation broth. Product yields of 96-99% and product purity of at least 96% can readily be obtained.

In view of the foregoing description taken with the examples, those skilled in the art will be able to practice the invention in various enablements without departing from the spirit and scope of the invention as defined in the appended claims.

## Claims

1. A simulated moving bed chromatography method for separating a basic amino acid from a feed solution comprising said basic amino acid and impurities, comprising:
(a) selecting a simulated moving bed chromatographic apparatus comprising one or more chromatographic columns connected in series, said column or columns containing a strong cation exchange chromatographic material comprising a functional group selected from the group consisting of sulfonates, alkylsulfonates, phenylsulfonates, alkylphenylsulfonates and mixtures thereof, and sequentially comprising a first desorbent port, an extract port, a feed port and a raffinate port; and
(b) simultaneously,
(i) contacting, through said feed port, said feed solution with the strong cation exchange chromatographic material equilibrated with an aqueous ammonia solution;
(ii) contacting, through said first desorbent port, a basic aqueous eluant with said strong cation exchange chromatographic material; and
(iii) withdrawing, through said extract port, an extract solution comprising said basic amino acid and a lower percentage by dry weight of said impurities than in said feed solution.

2. The method of claim 1, wherein said basic amino acid is lysine, arginine or histidine.

3. The method of any preceding claim, wherein said feed solution is a fermentation broth.

4. The method of claim 3, wherein said fermentation broth is a filtered fermentation broth.

5. The method of any preceding claim, wherein said strong cation exchange chromatographic material is a strong cation exchange resin.

6. The method of any preceding claim , wherein said strong cation exchange chromatographic material comprises a sulfonate.

7. The method of any preceding claim, wherein the simulated moving bed chromatographic apparatus further comprises a second desorbent port; a solution comprising water being withdrawn through said second desorbent port simultaneously with steps b(i)-b(iii).

8. The method of any preceding claim, wherein said basic aqueous eluant is an aqueous ammonia solution.

9. The method of claim 8, wherein said aqueous ammonia solution comprises between 0.5% and 10% ammonia, by weight.

10. The method of claim 8 or 9, wherein said aqueous ammonia solution comprises between 1% and 5% ammonia, by weight.

11. The method of any preceding claim, wherein, prior to contacting at (i), the pH of said feed solution is adjusted to a pH between 1 and 10 by the addition of an acidic or a basic material.

12. The method of claim 11, wherein the pH of said feed solution is adjusted to a pH between 2 and 7 by the addition of an acidic or a basic material.

13. The method of claim 12, wherein the pH of said feed solution is adjusted to a pH between 3 and 5 by the addition of an acidic or a basic material.

14. The method of any preceding claim, wherein the pH of said extract solution at (b)(iii) is at least one pH unit greater than the pH of said feed solution at (b)(i).

15. The method of claim 14, wherein the pH of said extract solution at (b)(iii) is at least 3 pH units greater than the pH of said feed solution at (b)(i).

## Patentansprüche

1. Simuliertes Wanderbett-Chromatografieverfahren für das Abtrennen einer basischen Aminosäure von einer Einspeisungslösung, umfassend die basische Aminosäure und Verunreinigungen, welches umfasst:
(a) Auswählen einer simulierten Wanderbett-Chromatografieapparatur, umfassend eine oder mehrere in Serie verbundene Chromatografiesäulen, wobei die Säule oder Säulen ein Chromatografiematerial für einen starken Kationenaustausch, umfassend eine funktionelle Gruppe ausgewählt aus der Gruppe bestehend aus Sulfonaten, Alkylsulfonaten, Phenylsulfonaten, Alkylphenylsulfonaten und Mischungen davon, enthalten, und umfassend sequentiell eine erste Öffnung für Desorptionsmittel, eine Öffnung für Extrakt, eine Öffnung für Einspeisung und eine Öffnung für Raffinat; und
(b) gleichzeitig
(i) Kontaktieren der Einspeisungslösung mit dem mit einer wässrigen Ammoniaklösung äquilibrierten Chromatografiematerial für einen starken Kationenaustausch durch die Öffnung für Einspeisung;
(ii) Kontaktieren eines basischen wässrigen Elutionsmittels mit dem Chromatografiematerial für einen starken Kationenaustausch durch die erste Öffnung für Desorptionsmittel; und
(iii) Entnehmen einer Extraktlösung, umfassend die basische Aminosäure und einen niedrigeren Prozentsatz, bezogen auf das Trockengewicht, an den Verunreinigungen als in der Einspeisungslösung, durch die Öffnung für Extrakt.

2. Verfahren nach Anspruch 1, bei dem die basische Aminosäure Lysin, Arginin oder Histidin ist.

3. Verfahren nach einem der vorangegangenen Ansprüche, bei dem die Einspeisungslösung eine Fermentationsbrühe ist.

4. Verfahren nach Anspruch 3, bei dem die Fermentationsbrühe eine gefilterte Fermentationsbrühe ist.

5. Verfahren nach einem der vorangegangenen Ansprüche, bei dem das Chromatografiematerial für einen starken Kationenaustausch ein Harz für einen starken Kationenaustausch ist.

6. Verfahren nach einem der vorangegangenen Ansprüche, bei dem das Chromatografiematerial für einen starken Kationenaustausch ein Sulfonat umfasst.

7. Verfahren nach einem der vorangegangenen Ansprüche, bei dem die simulierte Wanderbett-Chromatografieapparatur weiter eine zweite Öffnung für Desorptionsmittel umfasst und bei dem eine Wasser umfassende Lösung durch die zweite Öffnung für Desorptionsmittel gleichzeitig mit den Schritten b(i)-b(iii) entnommen wird.

8. Verfahren nach einem der vorangegangenen Ansprüche, bei dem das basische wässrige Elutionsmittel eine wässrige Ammoniaklösung ist.

9. Verfahren nach Anspruch 8, bei dem die wässrige Ammoniaklösung zwischen 0,5 % und 10 % Ammoniak, bezogen auf das Gewicht, enthält.

10. Verfahren nach Anspruch 8 oder 9, bei dem die wässrige Ammoniaklösung zwischen 1 % und 5 % Ammoniak, bezogen auf das Gewicht, enthält.

11. Verfahren nach einem der vorangegangenen Ansprüche, bei dem vor dem Kontaktieren bei (i) der pH der Einspeisungslösung auf einen pH zwischen 1 und 10 durch Zugabe von saurem oder basischem Material eingestellt wird.

12. Verfahren nach Anspruch 11, bei dem der pH der Einspeisungslösung auf einen pH zwischen 2 und 7 durch Zugabe von saurem oder basischem Material eingestellt wird.

13. Verfahren nach Anspruch 12, bei dem der pH der Einspeisungslösung auf einen pH zwischen 3 und 5 durch Zugabe von saurem oder basischem Material eingestellt wird.

14. Verfahren nach einem der vorangegangenen Ansprüche, bei dem der pH der Extraktlösung bei (b) (iii) wenigstens eine pH-Einheit größer als der pH der Einspeisungslösung bei (b) (i) ist.

15. Verfahren nach Anspruch 14, bei dem der pH der Extraktlösung bei (b) (iii) wenigstens 3 pH-Einheiten größer als der pH der Einspeisungslösung bei (b) (i) ist.

## Revendications

1. Procédé de chromatographie à lit mobile simulé pour séparer un acide aminé de base d'une solution de charge comprenant ledit acide aminé de base et des impuretés, comprenant :
(a) le choix d'un appareil chromatographique à lit mobile simulé comprenant une ou plusieurs colonnes chromatographiques raccordées en série, la ou lesdites colonnes contenant un matériau chromatographique d'échange cationique fort comprenant un groupement fonctionnel choisi dans le groupe constitué des sulfonates, des alkylsulfonates, des phénylsulfonates, des alkylphénylsulfonates et de leurs mélanges, et comprenant en séquence un premier orifice de désorbant, un orifice d'extrait, un orifice de charge et un orifice de raffinat ; et
(b) simultanément,
(i) la mise en contact, par ledit orifice de charge, de ladite solution de charge avec le matériau chromatographique d'échange cationique fort ;
(ii) la mise en contact, par ledit premier orifice de désorbant, d'un éluant aqueux de base avec ledit matériau chromatographique d'échange cationique fort ; et
(iii) le retrait, par ledit orifice d'extrait, d'une solution d'extrait comprenant ledit acide aminé de base et un pourcentage en poids à sec desdites impuretés inférieur à celui de ladite solution de charge.

2. Procédé selon la revendication 1, dans lequel ledit acide aminé de base est la lysine, l'arginine ou l'histidine.

3. Procédé selon l'une quelconque des revendications précédentes, dans lequel ladite solution de charge est un bouillon de fermentation.

4. Procédé selon la revendication 3, dans lequel ledit bouillon de fermentation est un bouillon de fermentation filtré.

5. Procédé selon l'une quelconque des revendications précédentes, dans lequel ledit matériau chromatographique d'échange cationique fort est une résine d'échange cationique forte.

6. Procédé selon l'une quelconque des revendications précédentes, dans lequel ledit matériau chromatographique d'échange cationique fort comprend un sulfonate.

7. Procédé selon l'une quelconque des revendications précédentes, dans lequel l'appareil chromatographique à lit mobile simulé comprend en outre un deuxième orifice de désorbant ; une solution comprenant de l'eau étant extraite par ledit deuxième orifice de désorbant simultanément avec les étapes b(i)-b(iii).

8. Procédé selon l'une quelconque des revendications précédentes, dans lequel ledit éluant aqueux de base est une solution d'ammoniaque.

9. Procédé selon la revendication 8, dans lequel ladite solution d'ammoniaque comprend entre 0,5 % et 10 % en poids d'ammoniac.

10. Procédé selon la revendication 8 ou 9, dans lequel ladite solution d'ammoniaque comprend entre 1 % et 5 % en poids d'ammoniac.

11. Procédé selon l'une quelconque des revendications précédentes, dans lequel, avant mise en contact en (i), le pH de ladite solution de charge est ajusté à un pH entre 1 et 10 par addition d'un matériau acide ou d'un matériau basique.

12. Procédé selon la revendication 11, dans lequel le pH de ladite solution de charge est ajusté à un pH entre 2 et 7 par addition d'un matériau acide ou d'un matériau basique.

13. Procédé selon la revendication 12, dans lequel le pH de ladite solution de charge est ajusté à un pH de 3 à 5 par addition d'un matériau acide ou d'un matériau basique.

14. Procédé selon l'une quelconque des revendications précédentes, dans lequel le pH de ladite solution d'extrait en (b)(iii) est supérieur d'au moins une unité de pH au pH de ladite solution de charge en (b) (i) .

15. Procédé selon la revendication 14, dans lequel le pH de ladite solution d'extrait en (b)(iii) est supérieur d'au moins 3 unités de pH au pH de ladite solution de charge en (b)(i).
